# EUROPEAN PATENT APPLICATION

(11) **EP 3 991 794 A1**
(43) Date of publication of application: **04.05.2022**
(21) Application number: 20831792.5
(22) Date of filing: 22.06.2020
(51) Int. Cl.: A61P 17/00, A61P 17/04, A61Q 19/00, A61K 8/34, A61K 8/37, A61K 8/44, A61K 8/46, A61K 8/55, A61K 8/63, A61K 47/10, A61K 47/14, A61K 47/18, A61K 47/20, A61K 47/24, A61K 31/56

(54) **EXTERNAL PREPARATION FOR SKIN**

(30) Priority: 27.06.2019 JP 2019120114
(71) Applicant: Kao Corporation, Chuo-ku Tokyo 103-8210 (JP)
(72) Inventor: KINUGASA, Yoshinori, Tokyo 131-8501 (JP); MATSUOKA, Megumi, Tokyo 131-8501 (JP); WATANABE, Manabu, Tokyo 131-8501 (JP)
(74) Representative: Hoffmann Eitle
(86) International application number: PCT/JP2020/024402
(87) International publication number: WO 2020/262299

(57) **Abstract**

An external skin preparation comprises the following components (A) to (E), wherein the mass ratio of the component (A) to the component (B), (A)/(B), is from 0.3 to 2.0, wherein the content of the component (C) is from 0.1 to 1 mass%, and wherein the preparation has a pH at 25°C of from 4.0 to 7.0: (A) one or more selected from the group consisting of aliphatic alcohols having from 14 to 20 carbon atoms; (B) two or more selected from the group consisting of polyhydric alcohol fatty acid ester-type nonionic surfactants; (C) one or more selected from the group consisting of N-acyl amino acids, N-acyl taurine, salts thereof, and phospholipids; (D) one or more selected from the group consisting of steroidal anti-inflammatory drugs, non-steroidal anti-inflammatory drugs, and heparinoids
; and (E) water.

## Description

### Technical Field

The present invention relates to an external skin preparation.

### Background Art

As main causes of inflammatory skin disease, in particular, of atopic dermatitis, biological overreaction to an antigen (allergen), i.e., immunological abnormalities and abnormalities in the barrier function of the skin are known. In normal skin, the skin moisture amount is maintained constant in the horny layer by natural moisturizing factors, such as sebum, amino acids, and urea, and keratinocyte intercellular lipids, such as ceramide and fatty acid, and the pH is also maintained slightly acidic. In contrast, in atopic dermatitis, it is known that these factors in the horny layer are decreased, the structural abnormalities of the horny layer are caused, the barrier function of the skin is reduced or destroyed, and also the pH is increased. Consequently, moisture evaporation from inside the skin is accelerated, the moisture amount in the horny layer is also decreased, and allergens from outside the skin are likely to invade. Accordingly, in treatment of atopic dermatitis, a problem is that even if the inflammation is improved once by a drug, the conditions will return due to permeated allergens when the external application is stopped.

Accordingly, a large number of proposals have been made as external skin preparations so far for the purpose of improving the barrier function of the skin. For example, a skin cosmetic comprising an intercellular lipid component, a moisturizing component, and a phospholipid (Patent Literature 1), an external skin preparation comprising N-acyl glutamic acid diester and pyrrolidonecarboxylic acid-modified dimethylpolysiloxane (Patent Literature 2), and an external preparation composition comprising a specific diamide derivative and a keratinocyte intercellular lipid component (Patent Literature 3) are mentioned.

### Citation List

### Patent Literature

[Patent Literature 1] JP-A-2004-168763
[Patent Literature 2] JP-A-2007-210892
[Patent Literature 3] JP-A-2002-332208

### Summary of Invention

The present invention provides an external skin preparation comprising the following components (A) to (E), wherein the mass ratio of the component (A) to the component (B), (A)/(B), is from 0.3 or more and 2.0 or less, wherein the content of the component (C) is from 0.1 mass% or more and 1 mass% or less, and wherein the preparation has a pH at 25°C of 4.0 or more and less than 7.0:
(A) one or more selected from the group consisting of aliphatic alcohols having 14 or more and 20 or less carbon atoms;
(B) two or more selected from the group consisting of polyhydric alcohol fatty acid ester-type nonionic surfactants;
(C) one or more selected from the group consisting of N-acyl amino acids, N-acyl taurine, salts thereof, and phospholipids;
(D) one or more selected from the group consisting of steroidal anti-inflammatory drugs, non-steroidal anti-inflammatory drugs, and heparinoids; and
(E) water.

### Detailed Description of Invention

Conventional external skin preparations or skin cosmetics, such as those disclosed in Patent Literatures 1 to 3, improve the barrier function of the skin, but do not have a function of reducing the contact and permeation of allergens to the skin. Accordingly, the present invention relates to an external skin preparation that not only improves the conditions of skin diseases but also enhances the barrier function of the skin and further can suppress the contact and permeation of allergens to the skin, while stably maintaining the emulsion state and the included drug at a pH in a weakly acidic region.

The present inventors have found that when a specific medicinal component is added to a base prescription of a combination of a higher alcohol, two or more specific nonionic surfactants, a phospholipid or a specific anionic surfactant, and water at a specific amount ratio, the smoothness and spreading property at the time of application at a pH in a weakly acidic region are excellent, the medicinal component is stably retained and the inflammatory state of a skin disease is effectively improved by forming a dry coating film having a uniform lamellar structure on the skin after the application, and allergen permeation that causes conditions to worsen again can be suppressed, and the present invention was accomplished.

The external skin preparation of the present invention can improve the conditions of skin diseases, also can enhance the barrier function of the skin, retain appropriate moisture permeability, and further suppress the contact and permeation of allergens to the skin, and also have excellent smoothness and spreading property at the time of application.

### [Component (A): aliphatic alcohol having from 14 to 20 carbon atoms]

The aliphatic alcohol having 14 or more and 20 or less carbon atoms as the component (A) is preferably a linear aliphatic alcohol and is preferably a saturated aliphatic alcohol. Examples of the component (A) include myristyl alcohol, cetyl alcohol, stearyl alcohol, and arachyl alcohol.

As the component (A), any of them may be used alone or in combination of two or more, but from the viewpoint of the storage stability of an emulsion state, it is preferable to use a combination of two or more compounds with different numbers of carbon atoms. Furthermore, from the same viewpoint, in the component (A), the mass ratio of short-chain (having 14 or more and less than 18 carbon atoms) aliphatic alcohol (a1) to long-chain (having 18 or more and 20 or less carbon atoms) aliphatic alcohol (a2), (a1)/(a2), is preferably 0.1 or more, more preferably 0.6 or more, and further preferably 1.0 or more and is preferably 7.0 or less, more preferably 5.0 or less, and further preferably 3.0 or less.

The content of the component (A) in the external skin preparation of the present invention is preferably 0.3 mass% or more, more preferably 0.5 mass% or more, and further preferably 0.8 mass% or more from the viewpoint of suppressing allergen permeation and is preferably 3.0 mass% or less, more preferably 2.5 mass% or less, and further preferably 2.0 mass% or less from the viewpoint of storage stability of the drug.

### [Component (B): two or more polyhydric alcohol fatty acid ester-type nonionic surfactants]

Examples of the component (B) include sucrose fatty acid ester, glycerin fatty acid ester, polyglycerin fatty acid ester, sorbitan monofatty acid, polyoxyethylene sorbitan fatty acid ester, polyoxyethylene sorbit fatty acid ester, polyoxyethylene glycerin fatty acid ester, polyoxyethylene fatty acid ester, and polyoxyethylene hydrogenated castor oil. Two or more thereof are used in combination. The combination of two or more polyhydric alcohol fatty acid esters is preferably a combination of one or more polyhydric alcohol fatty acid esters (b1) having an HLB of 3 or more and 7 or less, more preferably 4 or more and 6 or less, and one or more polyhydric alcohol fatty acid esters (b2) having an HLB of 11 or more and 16, more preferably 13 or more and 15 or less, from the viewpoint of forming a dry coating film having a lamellar structure. In addition, from the same viewpoint, the mass ratio thereof, (b1)/(b2), is preferably 0.25 or more, more preferably 0.4 or more, and further preferably 0.6 or more and is preferably 4.0 or less, more preferably 2.0 or less, and further preferably 1.0 or less.

The content of the component (B) in the external skin preparation of the present invention is preferably 0.5 mass% or more, more preferably 1.0 mass% or more, and further preferably 1.5 mass% or more from the viewpoint of the storage stability of an emulsion state and is preferably 5.0 mass% or less, more preferably 4.0 mass% or less, and further preferably 3.0 mass% or less from the viewpoint of smoothness at the time of application.

The mass ratio of the component (A) to the component (B), (A)/(B), in the external skin preparation of the present invention is 0.3 or more, preferably 0.35 or more, and more preferably 0.4 or more from the viewpoint of suppressing allergen permeation and is 2.0 or less, preferably 1.5 or less, more preferably 1.0 or less, and further preferably 0.8 or less from the viewpoint of good spreading property at the time of application.

### [Component (C): N-acyl amino acids, N-acyl taurine, salts thereof, and phospholipids]

The component (C) is a component providing an allergen permeation-suppressing function to the dry coating film having a lamellar structure formed on the skin.

The acyl groups of the acyl amino acids and acyl taurine in the component (C) are preferably those derived from fatty acids with saturated or unsaturated linear or branched chains or a mixture of these fatty acids, more preferably those derived from linear fatty acids or a mixture of linear fatty acids from the viewpoint of the storage stability of an emulsion state, and the number of carbon atoms thereof is preferably 6 or more, more preferably 10 or more, and further preferably 12 or more and is preferably 22 or less, more preferably 20 or less, and further preferably 18 or less. Such an acyl group is preferably one or more selected from the group consisting of a capryloyl group, a lauroyl group, a myristoyl group, a palmitoyl group, a stearoyl group, and a cocoyl group from the viewpoint of reducing the skin irritation and may be an acyl group derived from animal or vegetable oil, such as palm oil.

The amino acid portion of the acyl amino acids is preferably glutamic acid or aspartic acid from the viewpoint of storage stability of a drug. Here, the glutamic acid may be D-form, L-form, or a mixture of D-form and L-form and is preferably L-form. Specifically, such acyl amino acid(s) is preferably one or more selected from the group consisting of N-stearoyl glutamic acid, N-lauroyl glutamic acid, N-myristoyl glutamic acid, N-cocoyl glutamic acid, N-palm fatty acid glutamic acid, and N-lauroyl aspartic acid from the viewpoint of the storage stability of an emulsion state and is more preferably one or more selected from the group consisting of N-stearoyl glutamic acid and N-palm fatty acid glutamic acid.

The acyl taurine is preferably one or more selected from the group consisting of coconut oil fatty acid methyltaurine, N-caproyl methyltaurine, N-lauroyl methyltaurine, N-myristoyl methyltaurine, N-palmitoyl methyltaurine, N-stearoyl methyltaurine, and N-oleoyl methyltaurine from the viewpoint of the storage stability of an emulsion state and is more preferably N-stearoyl methyltaurine.

Examples of salts of these acyl amino acids and acyl taurine include alkali metal salts, such as a sodium salt and a potassium salt; alkaline earth metal salts, such as a calcium salt and a magnesium salt; other metal salts, such as an aluminum salt and a zinc salt; an ammonium salt; organic amine salts, such as a monoethanolamine salt, a diethanolamine salt, and a triethanolamine salt; and basic amino acid salts, such as an arginine salt, a lysine salt, a histidine salt, and an ornithine salt. These salts may be used alone or in combination of two or more. In particular, from the viewpoint of the storage stability of an emulsion state, the salts of the acyl amino acids and the acyl taurine are preferably alkali metal salts and more preferably sodium salts.

Examples of the phospholipids as the component (C) include glycerophospholipids, such as lecithin, hydrogenated lecithin, lecithin hydroxide, phosphatidylethanolamine, phosphatidylserine, phosphatidylcholine, phosphatidylinositol, phosphatidylglycerol, and cardiolipin; and sphingophospholipids, such as sphingomyelin, cerebroside, and ganglioside. Among these phospholipids, from the viewpoint of suppressing allergen permeation, hydrogenated lecithin is preferable, and soybean hydrogenated lecithin is more preferable.

Among the specific compounds above, the component (C) is preferably sodium N-acyl glutamate, sodium N-acyl methyltaurine, or a compound classified in hydrogenated lecithin from the viewpoint of the storage stability of an emulsion state. As the component (C) above, any of them can be used alone or in combination of two or more. The content of the component (C) in the external skin preparation of the present invention is 0.1 mass% or more, preferably 0.2 mass% or more, and more preferably 0.3 mass% or more from the viewpoint of suppressing allergen permeation and improving the storage stability of an emulsion state and is 1.0 mass% or less, preferably 0.8 mass% or less, and more preferably 0.6 mass% or less from the viewpoint of smoothness at the time of application and suppressing allergen permeation.

The mass ratio of the component (A) to the component (C), (A)/(C), in the external skin preparation of the present invention is preferably 1.0 or more, more preferably 1.5 or more, and further preferably 2.0 or more from the viewpoint of suppressing allergen permeation and is preferably 15 or less, more preferably 5.0 or less, and further preferably 3.0 or less from the viewpoint of the storage stability of an emulsion state.

### [Component (D): steroidal anti-inflammatory drug, non-steroidal anti-inflammatory drug, and heparinoid]

Examples of the steroidal anti-inflammatory drug in the component (D) include clobetasol propionic acid ester, diflorasone acetic acid ester, mometasone furancarboxylic acid ester, betamethasone butyric acid ester propionic acid ester, fluocinonide, betamethasone dipropionic acid ester, difluprednate, amcinonide, diflucortolone valeric acid ester, hydrocortisone butyrate propionate, deprodone propionic acid ester, dexamethasone propionic acid ester, dexamethasone valeric acid ester, betamethasone valeric acid ester, beclomethasone propionic acid ester, fluocinolone acetonide, prednisolone valeric acid ester acetic acid ester, triamcinolone acetonide, alclometasone propionic acid ester, clobetasone butyric acid ester, hydrocortisone butyric acid ester, prednisolone, dexamethasone acetic acid ester, hydrocortisone acetic acid ester, hydrocortisone valeric acid ester, dexamethasone metasulfobenzoate sodium, prednisolone valeric acid ester, dexamethasone, and hydrocortisone.

Examples of the non-steroidal anti-inflammatory drug in the component (D) include ufenamate, ibuprofen piconol, actarit, acemetacin, ampiroxicam, amfenac, ibuprofen, indomethacin, etodolac, ketoprofen, zaltoprofen, diclofenac, sulindac, celecoxib, tiaprofenic acid, tenoxicam, naproxen, piroxicam, felbinac, pranoprofen, flurbiprofen, mefenamic acid, medicoxib, meloxicam, mofezolac, rofecoxib, loxoprofen, lobenzarit, lornoxicam, and salts thereof. Among these drugs, one or more selected from the group consisting of ufenamate and ibuprofen piconol are preferable from the viewpoint of storage stability of a drug.

The content of the component (D) in the external skin preparation is preferably 0.025 mass% or more, more preferably 0.05 mass% or more, and further preferably 0.1 mass% or more from the viewpoint of the effectiveness of a drug and is preferably 6.5 mass% or less, more preferably 6.0 mass% or less, and further preferably 5.5 mass% or less from the viewpoint of the storage stability of an emulsion state.

In more detail, when the component (D) is a steroidal anti-inflammatory drug, the content thereof is, from the same viewpoint as above, preferably 0.025 mass% or more, more preferably 0.05 mass% or more, and further preferably 0.10 mass% or more and preferably 1.0 mass% or less, more preferably 0.5 mass% or less, and further preferably 0.3 mass% or less. When the component (D) is a non-steroidal anti-inflammatory drug, the content thereof is, from the same viewpoint as above, preferably 0.5 mass% or more, more preferably 2.5 mass% or more, and further preferably 4.5 mass% or more and preferably 6.5 mass% or less, more preferably 6.0 mass% or less, and further preferably 5.5 mass% or less. When the component (D) is a heparinoid, the content thereof is, from the same viewpoint as above, preferably 0.01 mass% or more, more preferably 0.1 mass% or more, and further preferably 0.3 mass% or more and preferably 3.0 mass% or less, more preferably 1.0 mass% or less, and further preferably 0.5 mass% or less.

### [Component (E): water]

The content of the component (E) in the external skin preparation of the present invention is preferably 50 mass% or more, more preferably 60 mass% or more, and further preferably 70 mass% or more from the viewpoint of good spreading property at the time of application and is preferably 95 mass% or less, more preferably 90 mass% or less, and further preferably 85 mass% or less from the viewpoint of suppressing allergen permeation.

### [Component (F): ceramide]

The external skin preparation of the present invention can comprise a ceramide as the component (F) for further improving the barrier function of the skin.

As the ceramide, one or more selected from the group consisting of natural ceramides and pseudo ceramides are mentioned. Examples of the natural ceramide include ceramide types 1 to 7 obtained by amidation of sphingosine, dihydrosphingosine, phytosphingosine, or sphingadienine and also include N-alkyl forms (for example, N-methyl forms) thereof. Examples of the pseudo ceramide include (N-hexadecyloxyhydroxypropyl)-N-hydroxyhexadecanamide, (N-hexadecyloxyhydroxypropyl)-N-hydroxydecanamide, N-[2-(2,3-dihydroxypropyloxy)-3-hexadecyloxypropyl]-N-3-methoxypropyltetradecanamide.

The content of the component (F) in the external skin preparation of the present invention is preferably 1.0 mass% or more, more preferably 1.5 mass% or more, and further preferably 2.0 mass% or more from the viewpoint of further improving the barrier function of the skin and is preferably 6.0 mass% or less, more preferably 5.0 mass% or less, and further preferably 4.0 mass% or less from the viewpoint of good spreading property at the time of application.

### [pH]

The pH of the external skin preparation of the present invention is 7.0 or less, preferably 6.5 or less, and more preferably 6.0 or less from the viewpoint of adjustment to normal skin pH environment and the viewpoint of improving the stability of the component (D) and is 4.0 or more, preferably 4.2 or more, and more preferably 4.4 or more from the viewpoint of reducing the skin irritation.

When the external skin preparation of the present invention comprises a steroidal anti-inflammatory drug as the component (D), the pH thereof is preferably 5.5 or less, more preferably 5.0 or less, and further preferably 4.8 or less from the viewpoint of the stability of the component (D) and is preferably 4.0 or more, more preferably 4.2 or more, and further preferably 4.4 or more from the viewpoint of the storage stability of an emulsion state.

When the external skin preparation of the present invention comprises a non-steroidal anti-inflammatory drug as the component (D), the pH thereof is preferably 7.0 or less, more preferably 6.5 or less, and further preferably 6.0 or less from the viewpoint of the storage stability of an emulsion state and is 4.0 or more, more preferably 4.5 or more, and further preferably 5.0 or more from the same viewpoint as above.

When the external skin preparation of the present invention comprises a heparinoid as the component (D), the pH thereof is preferably 7.0 or less, more preferably 6.5 or less, and further preferably 6.0 or less from the viewpoint of the storage stability of an emulsion state and is preferably 4.0 or more, more preferably 4.5 or more, and further preferably 5.0 or more from the same viewpoint as above.

### [Additional components]

The external skin preparation of the present invention can appropriately include, for example, oil other than the component (A), such as hydrocarbon oil, ether oil, ester oil, silicone oil, fluorine-based oil, wax, cholesterol derivatives, phytosterol derivatives, dipentaerythrite fatty acid esters, triglycerides, lanolin, lanosterol derivatives, and petrolatum; polyhydric alcohols, such as glycerin and propylene glycol; a thickener, a bactericide, a moisturizer, a wetting agent, a coloring agent, a preservative, a feel improver, a powder, an aroma chemical, an anti-inflammatory agent, a whitening agent, an antiperspirant, an UV absorber, and an antioxidant.

The external skin preparation of the present invention comprises the components (A) to (E) at predetermined ratios and thereby can form a dry coating film having a lamellar structure on a skin surface by being applied to the skin and evaporating the moisture. Incidentally, the lamellar structure can be verified, for example, by wide-angle X-ray diffraction or with a polarizing microscope.

Regarding the embodiments described above, preferred aspects of the present invention are further disclosed below.

<1>
   An external skin preparation comprising the following components (A) to (E), wherein the mass ratio of the component (A) to the component (B), (A)/(B), is 0.3 or more and 2.0 or less, wherein the content of the component (C) is 0.1 mass% or more and 1 mass% or less, and wherein the preparation has a pH at 25°C of 4.0 or more and less than 7.0:
   (A) one or more selected from the group consisting of aliphatic alcohols having 14 or more and 20 or less carbon atoms;
   (B) two or more selected from the group consisting of polyhydric alcohol fatty acid ester-type nonionic surfactants;
   (C) one or more selected from the group consisting of N-acyl amino acids, N-acyl taurine, salts thereof, and phospholipids;
   (D) one or more selected from the group consisting of steroidal anti-inflammatory drugs, non-steroidal anti-inflammatory drugs, and heparinoids; and
   (E) water.
<2>
   The external skin preparation according to <1>, wherein the component (A) is preferably one or more selected from the group consisting of myristyl alcohol, cetyl alcohol, stearyl alcohol, and arachyl alcohol.
<3>
   The external skin preparation according to <1> or <2>, wherein the component (A) is preferably a combination of two or more compounds with different numbers of carbon atoms.
<4>
   The external skin preparation according to <3>, wherein the mass ratio of aliphatic alcohol (a1) having 14 or more and less than 18 carbon atoms to aliphatic alcohols (a2) having 18 or more and 20 or less carbon atoms, (a1)/(a2), is preferably 0.1 or more, more preferably 0.6 or more, and further preferably 1.0 or more and is preferably 7.0 or less, more preferably 5.0 or less, and further preferably 3.0 or less.
<5>
   The external skin preparation according to any one of <1> to <4>, wherein the content of the component (A) is preferably 0.3 mass% or more, more preferably 0.5 mass% or more, and further preferably 0.8 mass% or more and is preferably 3.0 mass% or less, more preferably 2.5 mass% or less, and further preferably 2.0 mass% or less.
<6>
   The external skin preparation according to any one of <1> to <5>, wherein the component (B) is preferably a combination of two or more selected from the group consisting of sucrose fatty acid ester, glycerin fatty acid ester, polyglycerin fatty acid ester, sorbitan monofatty acid, polyoxyethylene sorbitan fatty acid ester, polyoxyethylene sorbit fatty acid ester, polyoxyethylene glycerin fatty acid ester, polyoxyethylene fatty acid ester, and polyoxyethylene hydrogenated castor oil.
<7>
   The external skin preparation according to any one of <1> to <6>, wherein the component (B) is preferably composed of one or more polyhydric alcohol fatty acid esters (b1) having an HLB of 3 or more and 7 or less, preferably 4 or more and 6 or less, and one or more polyhydric alcohol fatty acid esters (b2) having an HLB of 11 or more and 16, preferably 13 or more and 15 or less, and the mass ratio thereof, (b1)/(b2), is preferably 0.25 or more, more preferably 0.4 or more, and further preferably 0.6 or more and is preferably 4.0 or less, more preferably 2.0 or less, and further preferably 1.0 or less.
<8>
   The external skin preparation according to any one of <1> to <7>, wherein the content of the component (B) is preferably 0.5 mass% or more, more preferably 1.0 mass% or more, and further preferably 1.5 mass% or more and is preferably 5.0 mass% or less, more preferably 4.0 mass% or less, and further preferably 3.0 mass% or less.
<9>
   The external skin preparation according to any one of <1> to <8>, wherein the mass ratio of the component (A) to the component (B), (A)/(B), is preferably 0.35 or more and more preferably 0.4 or more and is preferably 1.5 or less, more preferably 1.0 or less, and further preferably 0.8 or less.
<10> The external skin preparation according to any one of <1> to <9>, wherein the acyl groups of the acyl amino acids and the acyl taurine in the component (C) are preferably those derived from fatty acids with saturated or unsaturated linear or branched chains or a mixture of these fatty acids, more preferably those derived from linear fatty acids or a mixture of linear fatty acids, in which the number of carbon atoms is preferably 6 or more, more preferably 10 or more, and further preferably 12 or more and is preferably 22 or less, more preferably 20 or less, and further preferably 18 or less.
<11>
   The external skin preparation according to any one of <1> to <10>, wherein the acyl amino acids or a salt thereof of the component (C) is preferably one or more selected from the group consisting of N-stearoyl glutamic acid, N-lauroyl glutamic acid, N-myristoyl glutamic acid, N-cocoyl glutamic acid, N-palm fatty acid glutamic acid, and N-lauroyl aspartic acid or salts thereof and is more preferably one or more selected from the group consisting of N-stearoyl glutamic acid and a salt thereof and N-palm fatty acid glutamic acid and a salt thereof.
<12>
   The external skin preparation according to any one of <1> to <11>, wherein the acyl taurine or a salt thereof of the component (C) is preferably one or more selected from the group consisting of coconut oil fatty acid methyltaurine, N-caproyl methyltaurine, N-lauroyl methyltaurine, N-myristoyl methyltaurine, N-palmitoyl methyltaurine, N-stearoyl methyltaurine, and N-oleoyl methyltaurine or salts thereof and is more preferably N-stearoyl methyltaurine or a salt thereof.
<13>
   The external skin preparation according to any one of <1> to <12>, wherein the phospholipid of the component (C) is preferably one or more selected from the group consisting of lecithin, hydrogenated lecithin, lecithin hydroxide, phosphatidylethanolamine, phosphatidylserine, phosphatidylcholine, phosphatidylinositol, phosphatidylglycerol, cardiolipin, sphingomyelin, cerebroside, and ganglioside, more preferably hydrogenated lecithin, and further preferably soybean hydrogenated lecithin.
<14>
   The external skin preparation according to any one of <1> to <13>, wherein the component (C) is preferably one or more selected from the group consisting of sodium N-acyl glutamate, sodium N-acyl methyltaurine, and hydrogenated lecithin.
<15>
   The external skin preparation according to any one of <1> to <14>, wherein the content of the component (C) is preferably 0.2 mass% or more and more preferably 0.3 mass% or more and is preferably 0.8 mass% or less and more preferably 0.6 mass% or less.
<16>
   The external skin preparation according to any one of <1> to <15>, wherein the mass ratio of the component (A) to the component (C), (A)/(C), is preferably 1.0 or more, more preferably 1.5 or more, and further preferably 2.0 or more and is preferably 15 or less, more preferably 5.0 or less, and further preferably 3.0 or less.
<17>
   The external skin preparation according to any one of <1> to <16>, wherein the component (D) is preferably one or more selected from the group consisting of prednisolone valeric acid ester acetic acid ester, prednisolone, hydrocortisone butyric acid ester, ufenamate, and heparinoid.
<18>
   The external skin preparation according to any one of <1> to <17>, wherein the content of the component (D) is preferably 0.025 mass% or more, more preferably 0.05 mass% or more, and further preferably 0.1 mass% or more and is preferably 6.5 mass% or less, more preferably 6.0 mass% or less, and further preferably 5.5 mass% or less.
<19>
   The external skin preparation according to any one of <1> to <18>, wherein the content of the component (E) is preferably 50 mass% or more, more preferably 60 mass% or more, and further preferably 70 mass% or more and is preferably 95 mass% or less, more preferably 90 mass% or less, and further preferably 85 mass% or less.
<20>
   The external skin preparation according to any one of <1> to <19>, further comprising a ceramide as a component (F).
<21>
   The external skin preparation according to <20>, wherein the content of the component (F) is preferably 1.0 mass% or more, more preferably 1.5 mass% or more, and further preferably 2.0 mass% or more and is preferably 6.0 mass% or less, more preferably 5.0 mass% or less, and further preferably 4.0 mass% or less.
<22>
   The external skin preparation according to any one of <1> to <21>, comprising a steroidal anti-inflammatory drug as the component (D), wherein the pH of the external skin preparation is preferably 5.5 or less, more preferably 5.0 or less, and further preferably 4.8 or less and is preferably 4.0 or more, more preferably 4.2 or more, and further preferably 4.4 or more.
<23>
   The external skin preparation according to any one of <1> to <21>, comprising a non-steroidal anti-inflammatory drug as the component (D), wherein the pH of the external skin preparation is preferably 7.0 or less, more preferably 6.5 or less, and further preferably 6.0 or less and is preferably 4.0 or more, more preferably 4.5 or more, and further preferably 5.0 or more.
<24>
   The external skin preparation according to any one of <1> to <21>, comprising a heparinoid as the component (D), wherein the pH of the external skin preparation is preferably 7.0 or less, more preferably 6.5 or less, and further preferably 6.0 or less and is preferably 4.0 or more, more preferably 4.5 or more, and further preferably 5.0 or more.

### Examples

### Examples 1 to 16 and Comparative Examples 1 to 5

External skin preparations shown in Tables 1 to 3 were prepared and were evaluated for "allergen permeation suppression", "drug stability", "emulsion stability", "smoothness at the time of application", and "spreading property at the time of application" according to the following methods and criteria.

### [Method for evaluating allergen permeation suppression]

A composition was applied on 12 mass% polyacrylamide gel (for SDS-PAGE) at 10 mg/cm² and was dried at room temperature for 24 hours. Subsequently, filter paper with a diameter of 6 mm was gently placed on the composition and was moistened with 20 µL of an aqueous dye solution (Fast Green FCF, 0.02 mg/20 mL). After leaving to stand under conditions of 32°C and 95% for 5 hours, the filter paper was taken off, and the dried composition was removed by washing with water. The amount of the dye permeated through the dried composition was measured using a densitometer 3 or 4 times for each composition, was expressed as a percentage with respect to the dye permeation amount, which was defined as 1, when the application was not performed, and was evaluated by the following criteria:
A: less than 20%;
B: 20% or more and less than 40%;
C: 40% or more and less than 60%;
D: 60% or more and less than 80%; and
E: 80% or more.

### [Method for evaluating storage stability of drug]

The content of each drug in a composition immediately after manufacturing was determined by comparison with a solution of a standard material of each drug (prednisolone valeric acid ester acetic acid ester, prednisolone, hydrocortisone butyric acid ester, ufenamate, or heparinoid) by liquid chromatography or ultraviolet-visible spectrophotometry. Subsequently, the composition was stored at 50°C for one month, the content of each drug in the composition after the storage was determined by comparison with a solution of the standard material as above, and the residual amount was expressed as a percentage with respect to the content in the composition immediately after the manufacturing defined as 1 and evaluated by the following criteria:
A: 95% or more;
B: 90% or more and less than 95%;
C: 85% or more and less than 90%;
D: 80% or more and less than 85%; and
E: less than 80%.

### [Method for evaluating storage stability of emulsion state]

The state (either creaming or aggregation) of a composition after storage at 50°C or -5°C for one month was visually verified and was evaluated by the following criteria:
A: no change;
B: slight change in the state;
C: clear change in the state;
D: partial separation or gelation; and
E: overall separation or gelation.

### [Method for evaluating smoothness at the time of application]

The smoothness when each emulsion composition was applied was sensorily evaluated by ten professional panelists and was judged by the following criteria:
A: seven to ten panelists evaluated as fine (good);
B: five or six panelists evaluated as fine (good);
C: three or four panelists evaluated as fine (good);
D: one or two panelists evaluated as fine (good); and
E: no panelist evaluated as fine (good).

### [Method for evaluating spreading property at the time of application]

The spreading property at the time of application of each emulsion composition was sensorily evaluated by ten professional panelists and was judged by the following criteria:
A: seven to ten panelists evaluated as fine (good);
B: five or six panelists evaluated as fine (good);
C: three or four panelists evaluated as fine (good);
D: one or two panelists evaluated as fine (good); and
E: no panelist evaluated as fine (good).

**[Table 1]**

| Component (mass%): contents are all active amounts | | | Example | Comparative Example | | | | |
|---|---|---|---|---|---|---|---|---|
| | | | 1 | 1 | 2 | 3 | 4 | 5 |
| (A) | (a1) | Cetyl alcohol | 0.6 | - | 0.6 | 0.6 | 0.6 | 0.6 |
| | (a2) | Stearyl alcohol | 0.4 | - | 0.4 | 0.4 | 0.4 | 0.4 |
| (A') | Behenyl alcohol | | - | 1 | - | - | - | - |
| (B) | (b2) | Polyoxyethylene(20) sorbitan monostearate HLB14.9 (^{∗}2) | 1.2 | 1.2 | 1.2 | - | 1.2 | 1.2 |
| | (b1) | Sorbitan monostearate HLB4.7 (^{∗}3) | 0.8 | 0.8 | - | 0.8 | 0.8 | 0.8 |
| (C) | Soybean hydrogenated lecithin (^{∗}4) | | 0.4 | 0.4 | 0.4 | 0.4 | - | - |
| (C') | Sodium cetyl sulfate | | - | - | - | - | - | 0.4 |
| (D) | Prednisolone valeric acid ester acetic acid ester | | 0.15 | 0.15 | 0.15 | 0.15 | 0.15 | 0.15 |
| (E) | Water | | Balance | Balance | Balance | Balance | Balance | Balance |
| Others | Succinic acid | | ^{∗}1 | ^{∗}1 | ^{∗}1 | ^{∗}1 | ^{∗}1 | ^{∗}1 |
| (a1)/(a2) | | | 1.50 | - | 1.50 | 1.50 | 1.50 | 1.50 |
| (b1)/(b2) | | | 0.67 | 0.67 | 0.00 | - | 0.67 | 0.67 |
| (A)/(B) | | | 0.50 | 0.50 | 0.83 | 1.25 | 0.50 | 0.50 |
| (A)/(C) | | | 2.50 | 2.50 | 2.50 | 2.50 | - | 2.50 |
| pH | | | 4.5 | 4.5 | 4.5 | 4.5 | 4.5 | 4.5 |
| Evaluation | Allergen permeation suppression | | A | D | D | E | C | E |
| | Storage stability of drug | | A | E | E | D | E | D |
| | Storage stability of emulsion state | | A | E | E | E | E | E |
| | Smoothness at the time of application | | A | D | D | D | D | D |
| | Spreading property at the time of application | | A | D | D | D | D | E |

| | | | | | | | | |
|---|---|---|---|---|---|---|---|---|
| ^{∗}1: Amount for pH adjustment ^{∗}2: RHEODOL TW-S120V, manufactured by Kao Corporation ^{∗}3: RHEODOL SP-S10V, manufactured by Kao Corporation ^{∗}4: COATSOME NC-21, manufactured by NOF Corporation | | | | | | | | |

**[Table 2]**

| Component (mass%): contents are all active amounts | | | Example | | | | | | | | | | |
|---|---|---|---|---|---|---|---|---|---|---|---|---|---|
| | | | 2 | 3 | 4 | 5 | 6 | 7 | 8 | 9 | 10 | 11 | 12 |
| (A) | (a1) | Cetyl alcohol | - | 0.6 | 0.6 | 0.6 | 0.6 | 0.6 | 0.6 | 0.6 | 0.6 | 2 | 0.6 |
| | (a2) | Stearyl alcohol | 0.8 | 0.4 | 0.4 | 0.4 | 0.4 | 0.4 | 0.4 | 0.4 | 0.4 | 1 | 0.4 |
| (B) | (b2) | Polyoxyethylene(20) sorbitan monostearate HLB14.9 (^{∗}2) | 1.2 | - | - | - | 1.2 | 1.2 | 1.2 | 1.2 | 1.2 | 1.2 | 1.2 |
| | | Polyoxyethylene hydrogenated castor oil (40E.O.) HLB12.5 (^{∗}5) | - | 1.2 | 1.2 | - | - | - | - | - | - | - | - |
| | | Decaglyceryl monostearate HLB12.0 (^{∗}6) | - | - | - | 1.2 | - | - | - | - | - | - | - |
| | (b1) | Sorbitan monostearate HLB4.7 (^{∗}3) | 0.8 | 0.8 | - | 0.8 | 0.8 | 0.8 | 0.8 | 0.8 | 0.8 | 0.8 | 0.8 |
| | | Glyceryl monostearate HLB4.0 (^{∗}7) | - | - | 0.8 | - | - | - | - | - | - | - | - |
| (C) | Soybean hydrogenated lecithin (^{∗}4) | | 0.4 | 0.4 | 0.4 | 0.4 | 0.1 | 0.2 | 1 | - | - | 0.3 | 0.4 |
| | Palm fatty acid sodium glutamate (^{∗}8) | | - | - | - | - | - | - | - | 0.4 | - | - | - |
| | N-Stearoyl-N-methyltaurine sodium (^{∗}9) | | - | - | - | - | - | - | - | - | 0.4 | - | - |
| (D) | Prednisolone valeric acid ester acetic acid ester | | 0.15 | 0.15 | 0.15 | 0.15 | 0.15 | 0.15 | 0.15 | 0.15 | 0.15 | 0.15 | 0.15 |
| (E) | Water | | Balance | Balance | Balance | Balance | Balance | Balance | Balance | Balance | Balance | Balance | Balance |
| (F) | N-(Hexadecyloxyhydroxypropyl)-N-hydroxyethylhexadecanamide | | - | - | - | - | - | - | - | - | - | - | 3 |
| Others | Succinic acid | | ^{∗}1 | ^{∗}1 | ^{∗}1 | ^{∗}1 | ^{∗}1 | ^{∗}1 | ^{∗}1 | ^{∗}1 | ^{∗}1 | ^{∗}1 | ^{∗}1 |
| (a1)/(a2) | | | 0 | 1.50 | 1.50 | 1.50 | 1.50 | 1.50 | 1.50 | 1.50 | 1.50 | 2.00 | 1.50 |
| (b1)/(b2) | | | 0.67 | 0.67 | 0.67 | 0.67 | 0.67 | 0.67 | 0.67 | 0.67 | 0.67 | 0.67 | 0.67 |
| (A)/(B) | | | 0.40 | 0.50 | 0.50 | 0.50 | 0.50 | 0.50 | 0.50 | 0.50 | 0.50 | 1.50 | 0.50 |
| (A)/(C) | | | 2.00 | 2.50 | 2.50 | 2.50 | 10.00 | 5.00 | 1.00 | 2.50 | 2.50 | 10.00 | 2.50 |
| pH | | | 4.5 | 4.5 | 4.5 | 4.5 | 4.5 | 4.5 | 4.5 | 4.5 | 4.5 | 4.5 | 4.5 |
| Evaluation | Allergen permeation suppression | | B | A | A | B | B | A | B | A | A | B | A |
| | Storage stability of drug | | B | A | A | A | A | A | A | A | B | B | A |
| | Storage stability of emulsion state | | B | B | A | B | C | B | A | B | A | B | A |
| | Smoothness at the time of application | | A | A | A | A | A | A | B | A | A | B | A |
| | Spreading property at the time of application | | A | A | A | B | B | A | B | A | A | A | A |

| | | | | | | | | | | | | | |
|---|---|---|---|---|---|---|---|---|---|---|---|---|---|
| ^{∗}1: Amount for pH adjustment ^{∗}6: NIKKOL DECAGLYN 1-SV, manufactured by Nikko Chemicals Co., Ltd. ^{∗}2: RHEODOL TW-S120V, manufactured by Kao Corporation ^{∗}7: NIKKOL MGS-AMV, manufactured by Nikko Chemicals Co., Ltd. ^{∗}3: RHEODOL SP-S10V, manufactured by Kao Corporation ^{∗}8: AMISOFT GS-11P, manufactured by AJINOMOTO Co., Inc. ^{∗}4: COATSOME NC-21, manufactured by NOF Corporation ^{∗}9: NIKKOL SMT, manufactured by Nikko Chemicals Co., Ltd. ^{∗}5: EMANON CH-40, manufactured by Kao Corporation | | | | | | | | | | | | | |

**{Table 3]**

| Component (mass%): contents are all active amounts | | | Example | | | |
|---|---|---|---|---|---|---|
| | | | 13 | 14 | 15 | 16 |
| (A) | (a1) | Cetyl alcohol | 0.6 | 0.6 | 0.6 | 0.6 |
| | (a2) | Stearyl alcohol | 0.4 | 0.4 | 0.4 | 0.4 |
| (B) | (b2) | Polyoxyethylene(20) sorbitan monostearate HLB14.9 (^{∗}2) | 1.2 | 1.2 | 1.2 | 1.2 |
| | (b1) | Sorbitan monostearate HLB4.7 (^{∗}3) | 0.8 | 0.8 | 0.8 | 0.8 |
| (C) | Soybean hydrogenated lecithin (^{∗}4) | | 0.4 | 0.4 | 0.4 | 0.4 |
| (D) | Prednisolone | | 0.25 | - | - | - |
| | Hydrocortisone butyric acid ester | | - | 0.05 | - | - |
| | Ufenamate | | - | - | 5 | - |
| | Heparinoid | | - | - | - | 0.3 |
| (E) | Water | | Balance | Balance | Balance | Balance |
| Others | Succinic acid | | ^{∗}1 | ^{∗}1 | ^{∗}1 | ^{∗}1 |
| (a1)/(a2) | | | 1.50 | 1.50 | 1.50 | 1.50 |
| (b1)/(b2) | | | 0.67 | 0.67 | 0.67 | 0.67 |
| (A)/(B) | | | 0.50 | 0.50 | 0.50 | 0.50 |
| (A)/(C) | | | 2.50 | 2.50 | 2.50 | 2.50 |
| pH | | | 4.5 | 4.5 | 5.5 | 5.5 |
| Evaluation | Allergen permeation suppression | | A | A | A | A |
| | Storage stability of drug | | A | A | A | A |
| | Storage stability of emulsion state | | A | A | A | A |
| | Smoothness at the time of application | | A | A | A | A |
| | Spreading property at the time of application | | A | A | A | A |

| | | | | | | |
|---|---|---|---|---|---|---|
| ^{∗}1: Amount for pH adjustment ^{∗}2: RHEODOL TW-S120V, manufactured by Kao Corporation ^{∗}3: RHEODOL SP-S10V, manufactured by Kao Corporation ^{∗}4: COATSOME NC-21, manufactured by NOF Corporation | | | | | | |

### Example 17

An external skin preparation of the following prescription was prepared, and the "allergen permeation suppression", "smoothness at the time of application", and "spreading property at the time of application" were evaluated according to the methods described above, and the results were "B", "B", and "B", respectively.

| | (mass%) |
|---|---|
| (a2) Stearyl alcohol | 3.0 |
| (b2) Polyoxyethylene (20) sorbitan monostearate | 0.9 |
| (b1) Sorbitan monostearate | 0.6 |
| (C) Soybean hydrogenated lecithin | 0.4 |
| (C) Sodium N-lauroyl glutamate | 0.1 |
| (D) Heparinoid | 0.3 |
| (F) N-(Hexadecyloxyhydroxypropyl)-N-hydroxyethylhexadecanamide | 3.0 |
| Squalane | 1.0 |
| Dimethylpolysiloxane | 3.0 |
| Methylparaben | 0.2 |
| Succinic acid amount for pH | adjustment |
| (E)Water | Balance |
| pH = 5.1 | |
| (a1)/(a2) = 0 | |
| (b1)/(b2) = 0.67 | |
| (A)/(B) = 2.00 | |
| (A)/(C) = 6.00 | |

## Claims

1. An external skin preparation comprising following components (A) to (E), wherein a mass ratio of the component (A) to the component (B), (A)/(B), is 0.3 or more and 2.0 or less, wherein a content of the component (C) is 0.1 mass% or more and 1 mass% or less, and wherein the preparation has a pH at 25°C of 4.0 or more and 7.0 or less:
(A) one or more selected from the group consisting of aliphatic alcohols having 14 or more and 20 or less carbon atoms;
(B) two or more selected from the group consisting of polyhydric alcohol fatty acid ester-type nonionic surfactants;
(C) one or more selected from the group consisting of N-acyl amino acids, N-acyl taurine, salts thereof, and phospholipids;
(D) one or more selected from the group consisting of steroidal anti-inflammatory drugs, non-steroidal anti-inflammatory drugs, and heparinoids; and
(E) water.

2. The external skin preparation according to claim 1, wherein the mass ratio of the component (A) to the component (B), (A)/(B), is 0.35 or more and 1.0 or less.

3. The external skin preparation according to claim 1 or 2, wherein a mass ratio of the component (A) to the component (C), (A)/(C), is 1.0 or more and 15 or less.

4. The external skin preparation according to any one of claims 1 to 3, wherein the component (B) is composed of one or more polyhydric alcohol fatty acid esters (b1) having an HLB of 3 or more and 7 or less and one or more polyhydric alcohol fatty acid esters (b2) having an HLB of 11 or more and 16, and a mass ratio thereof, (b1)/(b2), is 0.25 or more and 4.0 or less.

5. The external skin preparation according to any one of claims 1 to 4, wherein a content of the component (D) is 0.025 mass% or more and 6.5 mass% or less.

6. The external skin preparation according to any one of claims 1 to 5, wherein the component (C) is one or more selected from the group consisting of sodium N-acyl glutamate, sodium N-acyl methyltaurine, and hydrogenated lecithin.

7. The external skin preparation according to any one of claims 1 to 6, further comprising a ceramide as a component (F).
